# EUROPEAN PATENT SPECIFICATION

(11) **EP 0 844 861 B1**
(45) Date of publication and mention of the grant of the patent: **20.03.2002**
(21) Application number: 96929230.9
(22) Date of filing: 09.08.1996
(51) Int. Cl.: A61F 13/46, A61F 13/15

(54) **ABSORBENT ARTICLES**
ABSORBIERENDE GEGENSTÄNDE
ARTICLES ABSORBANTS

(30) Priority: 11.08.1995 GB 9516447; 01.12.1995 GB 9524570; 12.07.1996 GB 9614668
(43) Date of publication of application: 03.06.1998
(73) Proprietor: CAMELOT SUPERABSORBENTS LTD, Calgary, Alberta T2E 7L6 (CA)
(72) Inventor: DAN, Ervin, Calgary, Alberta T2R 1H3 (CA); SHIPLEY, Arthur, Roger, Prestatyn, Clwyd LL19 9PU (GB)
(74) Representative: Ritter, Stephen David
(86) International application number: EP9603530
(87) International publication number: WO9706765

(56) References cited:
- EP-A- 0 040 084
- WO-A-91/11161
- WO-A-92/11830
- WO-A-92/18078
- GB-A- 2 225 724
- GB-A- 2 266 465
- GB-A- 2 278 371
- US-A- 4 892 532

## Description

The present invention relates to absorbent articles. In particular, it relates to articles such as sanitary napkins and pads, incontinence garments and disposable diapers. In addition, the present invention relates to a water-management layer for an absorbent article.

As used herein, the term "water" when used alone or in the phrases "water-absorbing", "water-absorbent", "water-swellable" and "water-management" is understood to mean not only water but also aqueous media such as, in particular, electrolyte solutions such as body fluids.

Sanitary napkins, pads, incontinence garments and disposable diapers have been known for many years and much effort has been made to improve the functional efficiency of such articles to make them more absorbent, more comfortable to wear and less obtrusive to the wearer.

In general, such products have a core which includes a water-absorbent layer. This water-absorbent layer may be formed from any suitable water-absorbent material including wood pulp, rayon and tissue. Additionally, or alternatively, the layer may comprise any of the water-absorbing polymer compositions commonly known as superabsorbent polymers. Layered absorbent structures are disclosed in EP 0 040 084, GB 2 225 724A, WO 92/18078, US 4 892 532 and WO 92/11830.

A number of absorbent compositions have been developed which exhibit the capacity to be water-absorbing. Known compositions may be in any suitable form including powders, particles and fibers. US 3,954,721 and US 3,983,095, disclose preparations for derivatives of copolymers of maleic anhydride with at least one vinyl monomer in fibrous form. The fibrous copolymers are rendered hydrophillic and water-swellable by reaction with ammonia or an alkali metal hydroxide. US 3,810,468, discloses lightly cross-linked olefin-maleic anhydride copolymers prepared as substantially linear copolymers and then reacted with a diol or a diamine to introduce cross-linking. The resultant lightly cross-linked copolymers are treated with ammonia or an aqueous or alcohol solution of an alkali metal hydroxide. US 3,980,663, describes water-swellable absorbent articles made from carboxylic polyelectrolytes via cross-linking with glycerine diglycidyl ether.

European Published Application No. 0 268 498 describes a water-absorbent composition formed by causing a substantially linear polymer of water-soluble ethylenically unsaturated monomer blends comprising carboxylic and hydroxylic monomers to cross-link internally.

Further examples of water-absorbent compositions are those produced from a copolymer of an α,β unsaturated monomer having at least one pendant unit selected from a carboxylic acid group and derivatives thereof and a copolymerisable monomer. A proportion of the pendant units are present in the final copolymer as the free acid and a proportion as the salt of the acid. These copolymers are capable of being cross-linked, either internally or with a variety of cross-linking agents, to form the water-swellable composition. Examples of water-swellable compositions of this type can be found in US 4,616,063, 4,705,773, 4,731,067, 4,743,244, 4,788,237, 4,813,945, 4,880,868 and 4,892,533 and EP 0 272 074, 0 264 208 and 0 436 514.

Derivatives of carboxylic acid groups include carboxylic acid salt groups, carboxylic acid amide groups, carboxylic acid imide groups, carboxylic acid anhydride groups and carboxylic acid ester groups.

Other examples of water-absorbent compositions can be found in US 4,798,861, WO 93/17066, WO 93/255735, WO 93/24684, WO 93/12275, European Published Application Nos 0 401 044, 0 269 393, 0 326 382, 0 227 305, 0 101 253, 0 213 799, 0 232 121, 0 342 919, 0 233 014, 0 268 498 and 0 397 410, British Patent Application Nos 2 082 614, 2 022 505, 2 270 030, 2 269 602 and 2 126 591, U.S. Patent Nos 4,418,163, 4,418,163, 3,989,586, 4,332,917, 4,338,417, 4,420,588 and 4,155,957 and French Patent Application No. 2 525 121 herein by reference.
Water-absorbent material of the kinds referred to in the above-mentioned patents and applications may be in any suitable form including powder, particulate granular and fibers; the fibers may be straight or may be curled and/or crimped. Details of such curly/crimped fibers may be found in US 4,822,453, 4,888,453 5,462,793 and 4,898,462. In one alternative material, the water-absorbent polymer may be coated onto the whole or a part of the surface of other materials such as non-water-absorbent fibers. Details of one type of fibers of this type may be found in WO 96/15307.

Other kinds of water-absorbent materials may be used as, or as part of, the water-absorbing layer. Suitable materials include naturally occurring water-absorbent materials. One such water-absorbent material is starchy material such as that proposed by the US Department of Agriculture in 1969-1970. Peat moss may also be used a water-absorbent material. In this connection, reference may be made to US 5,477,627 5,429,242 5,374,260 4,992,324 4,676,871 4,573,988 4,560,372 4,540,454 4,537,590 and 4,226,237. Alginates have also been proposed as suitable water-absorbent material. One example of the use of such alginates is that suggested by Beghin/Kayserberg.

The water-absorbent layer may be of non-uniform configuration. For example, where the absorbent article is a feminine hygiene product or a diaper, the water-absorbent layer may be shaped such that it is thicker in the crotch region.

Whatever material is used for the absorbent layer, it is generally backed by a fluid-impervious backing sheet to protect clothing and the surrounding area from soiling and to prevent leakage of the body fluid which would cause embarrassment to the user. Any water-impervious backing material may be used.

The article generally has a water-permeable non-woven type cover-sheet which defines the surface of the article which will, in use, be in contact with the user. The cover sheet is intended to insulate the wearer from contact with water that has been absorbed into the core, thus the cover sheet should allow water to pass through it into the core but remain dry and soft to the touch. Any suitable material may be used as the cover-sheet. One example of a suitable cover-sheet is described in US 5,257,982.

As there is often a discharge of a substantial volume of water in a short time, the core may additionally include a distribution layer which is generally located above the water-absorbent layer. This distribution layer serves to wick the water away from the point of initial contact with the article and transport it to other parts of the water-absorbent layer. The use of a distribution layer of this type is advantageous in addressing the problem of pooling if a substantial volume of water is discharged in a short time and thereby gives the user a feeling of dryness. Examples of distribution layers include those described in EP 0 565 606, GB 2 266 465 and GB 2 278 371.

As the distribution layer serves to wick the water away from the point of initial contact, it is accepted that the water should be transported in the plane of the layer as quickly as possible and thus, distribution layers are generally made of hydrophillic material. The concept of speeding up the rate at which water is transported in the plane of the distribution layer is discussed in detail in EP 0 565 606 assigned to The Procter & Gamble Company in which the distribution layer forms part of the multiple layer absorbent core.

Whilst the use of distribution layers of this type go some way to reducing the effects of pooling, they do not satisfactorily overcome the problems of rewet once the water has been absorbed into the water-absorbent layer. Rewet is a measure of how dry the articles feel to the touch after water-absorption. This is generally measured under load of approximately 3-5kPa. Rewet is of particular importance in the applications to which the absorbent articles of the present invention are directed as if the article feels damp, the user will feel damp and uncomfortable.

The problem of rewet is particularly acute where the water-absorbent layer is formed from or includes the water-absorbent polymers described above, in particular where it is formed from fast absorbing water-absorbent polymers. Although the water-absorbent polymers described above can absorb a large volume of water and absorption can take place in a short time period, in some circumstances, the water-absorbent polymeric may become locally saturated because of the fast absorbency of the water-absorbent and may thereby feel damp to the touch.

We have now discovered that an alternative distribution layer may be provided which satisfactorily transports the water away from the initial point of contact and overcomes the aforementioned problems of rewet if the distribution layer is formed from material which slows down the rate at which the water is transported through the layer with respect to the rate at which the water is transported in the plane of the layer. The distribution layer will hereinafter be referred to as a water-management layer to distinguish it from conventional distribution layers.

Thus the present invention provides a multiple layer water-absorbent core suitable for use in an absorbent article comprising a water-absorbent layer and a water-management layer, which water management layer has an upper surface and is an apertured film or a hydrophobic non-woven material and which water-management layer is located above said water-absorbent layer and which water-management layer has fibers of from 15 to 50 mm in length located on its upper surface.

An hydrophobic layer has been found to be a particularly effective alone or as a component of a water-management layer. This discovery is particularly surprising since it would be expected that an hydrophobic water-management layer would reduce the ability of the layer to remove water from the point of contact.

The water-management layer suitable for use in conjunction with a water-absorbent layer in an absorbent article can act to reduce the exposure of the water-absorbent layer to water. That is to say that the rate of transport of the water through the layer is slower than in conventional distribution layers. This allows the water to spread through/along the layer before it passes through the layer.

By "exposure" we mean the time that elapses between the water being applied to the surface of the water-management layer and its entering the water-absorbent layer.

The material from which the water-management layer is preferably formed is a hydrophobic material or a material made hydrophobic by being treated with a suitable agent, for example a silicone.

The water-absorbent layer of the core preferably includes a water-absorbent polymeric composition which may be in the form of fibers.

Any material which allows the water to spread along the plane of the layer before it passes through the layer may be used. One suitable material from which the water-management layer may be formed is an apertured film. Examples of apertured films include those described in WO 94/28846, US 5,478,335, DE 959814, EP 0 598 970, US 4,806,303, US 4,626,254, US 4,634,440, US 5,180,620, US 5,387,209, US 5,449,352, IT 591405, US 4,895,749, US 5,078,710 and EP 0 195 113 and those described by Smith & Nephew, AET (under the trade name HERCULES), Johnson & Johnson (under the trade name RETICULON), Pollguf, Evial, Plastik, EPG, Avgul and Honshu.

A preferred apertured film is a plastics, preferably polypropylene, film having spaced holes. The polypropylene film may have from about 50 holes per square inch to about 250 holes per square inch, preferably 100 to 180, more preferably 130 to 150 holes per square inch. 140 holes per square inch may also be used.

In a particularly preferred embodiment, some or all of the holes have a three-dimensional structure, most preferably, some or all of the holes are fluted, ie funnel shaped. Fluted holes may be formed by any appropriate means. One suitable means is a method is which the polymeric sheet is placed over a grid having spaced holes and heating the sheet whilst applying a vacuum to the side of the grid remote from that of the polymeric sheet. As the sheet is heated it softens and material is pulled, by the force of the vacuum, through the holes until the flutes formed burst at their bases thereby forming the apertured film. One alternative means involves treating the polypropylene film with hot needles. The fluted holes are preferably configured such that the base of the apertures extend beneath the bottom surface of the plane of the film. Thus, when the apertured film is used in the absorbent article of the present invention the bottom of the fluted apertures preferably penetrate the water-absorbent layer. However, the flutes may also be oriented such that they are directed away from the water-absorbent layer in use.

In one alternative arrangement, the apertured film may comprise a plurality of sheets each having holes. The holes of each sheet may be of different diameters and configured such that when the sheets are laid one on another, the holes "line-up" to form an aperture of non-cylindrical, preferably fluted, cross section.

The apertured polymeric film may be corrugated.

One alternative suitable material for the water-management layer is a non-woven material that is made hydrophobic and thereby slows down the rate of transport of water through the layer which thereby forces lateral water-management of the water.

The non-woven material may be any suitable non-woven material that has been treated such that it becomes hydrophobic. Any means of making the non-woven material hydrophobic may be used. One example of a suitable treatment is spraying the material with a silicone composition. The non-woven material may be made by any suitable means from non-water-absorbent fibers. Suitable means include melt-blown and spun-bonded methods. By "non-water-absorbent" we mean that the fibers do not absorb to an appreciable extent. Suitable materials from which the non-woven material may be formed include natural or synthetic fibers such as cellulose, viscose, polyester, non-water-absorbent polymers of propylene, polyamide and ethylenepropylene copolymer fibers, and mixtures thereof and the like, with polyesters, polyethylene and polypropylene fibers being particularly preferred.

Additionally, or alternatively, the water-management layer, howsoever formed, may be or include arrangements sometimes known as "lateral movers" located on the upper surface of the layer. Suitable lateral movers include creped tissue, embossed tissue (additionally or alternatively, the water-management layer itself may be embossed), a "Burgeni" skin arrangement for example that described in US 2,925,260, under layers such as those described in EP 0 523 719, US 5,437,653, WO 92/09716 and US 5,415,640, the arrangement described in US 2,945,386 and the arrangements in WO 93/11725, US 5,411,497, US 5,425,725 and US 5,433,715.

The fibers located on the upper surface of the water-management layer may be individual fibers or a non-woven or woven mat of fibers. The fibers in the mat may be bonded. Without wishing to be bound by any particular theory it is believed that these fibers create a lofted area that behaves as capillaries for the lateral movement of the fluid. The fibers may be arranged such that they generally extend away from the water-management layer such that when in use in an absorbent article, the fibers are directed towards the cover sheet where present. The fibers may be arranged such that there are voids between the fibers. The fibers may, in one embodiment, be bonded to each other.

In a preferred arrangement there are grooves or channels formed between the fibers or between groups of fibers. The arrangement of the fibers such that there are grooves or channels present may be achieved by any suitable means. However, carding is particularly preferred. The grooves or channels preferably run substantially parallel to the longitudinal edge of the absorbent article. For example, where the absorbent article is a sanitary towel or pant liner, the grooves or channels will run lengthways along the pad.

In one alternative arrangement the fibers themselves may be grooved. In this connection, reference may be made to Eastman Chemicals 4DG fibers.

The fibers may be any suitable material and are preferably selected from rayon fibers, cellulose ester fibers, protein fibers, polyamide fibers, polyester fibers, polyvinyl fibers, polyolefin fibers, polyurethane fibers, aramid fibers, glass fibers and mixtures thereof. Particularly useful fibers are fibers having a hollow core such as the polyester, typically polyester terephthalate, fibers commercially available from E. I. DuPont de Nemours under the trade name HOLLOWFILL. Bicomponent fibers may also be used.

The fibers are from about 15 to about 50 mm in length but are preferably 20 to 35 mm in length and are more preferably 25 mm in length.

According to a further aspect of the present invention, there is provided an absorbent article including the multi-layered core of the present invention.

According to a further aspect of the present invention, there is provided an absorbent article comprising a liquid pervious cover sheet, a liquid impervious backsheet and the multi-layered core of the present invention wherein the cover sheet is joined to the backsheet to enclose the core. In a further aspect, the fibers may be arranged so that they extend in a direction substantially parallel to the longitudinal axis of the absorbent article.

The layers of the absorbent article are preferably bonded to adjacent layers. Thus, for example, the water-absorbent layer will be bonded to the water-management layer and the water-management layer will be bonded to the cover sheet. The fibers located on the upper surface of the water-management layer are preferably bonded to the water-management layer. Further, when the absorbent article has a cover sheet, the fibers are preferably bonded to the cover sheet using a sprayable adhesive which is preferably a hotmelt adhesive. Thus, the fibers may be bonded to the water-management layer, to the cover sheet, or to the water-management layer and the cover sheet. Where individual fibers are used, each fiber may be bonded individually. Where a mat of fibers is used, it may only be necessary to bond portions of the mat to the adjacent layers.

The adhesive may be applied such that it forms a pattern, wherein the spaces between the nodules of adhesive may form grooves and channels which may serve to direct the fluid laterally. Thus, an adhesive may be used as one alternative to, or in addition to, the fibers described above.

In addition to reducing the pooling effects discussed above, the water-management layer of the present invention may also act to reduce rewet. Without wishing to be bound by any theory, it is believed that the water-management layer of the present invention allows water to pass through it in a controlled manner as discussed above and then behaves as an enclosure to prevent water in the water-absorbent layer from coming into contact with the user.

A particularly preferred embodiment of the present invention will now be described, with reference to the accompanying drawings in which:
- Figure 1: is a schematic representation of a diaper including the water-management layer of the present invention; and
- Figure 2: is a schematic representation of one water-management layer of the present invention.

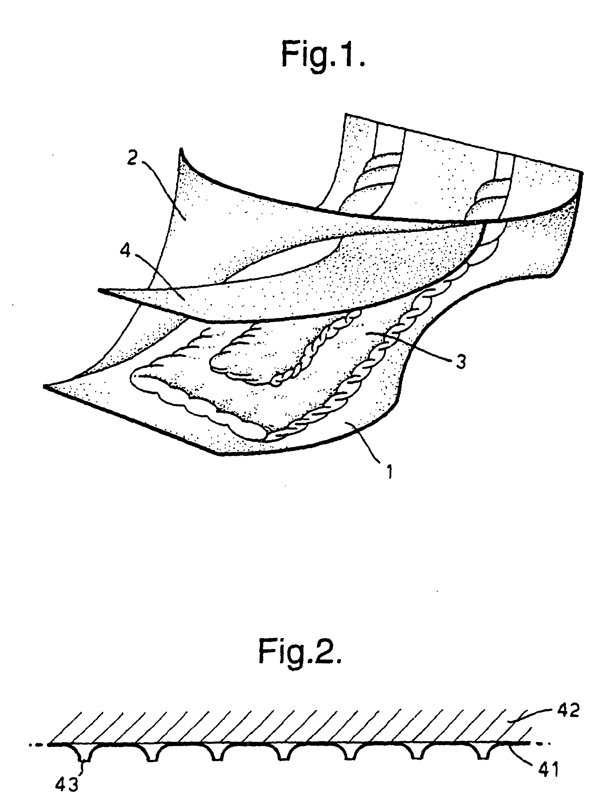

The diaper as illustrated in Figure 1 is of simple construction for ease of understanding. It will be understood that the diaper may be of any configuration and may include features to aid in the fitting of the diaper to the baby, including elastication and closure tabs. The diaper comprises a polyethylene backsheet 1 and a non-woven cover stock 2 which are joined around their peripheries in conventional manner to enclose a three dimensional water-absorbent layer 3. The water-absorbent layer may be of any suitable arrangement and may consist of, for example, bulking material such as wood fluff and water-absorbent material.

The water-absorbent material is preferably water-absorbent fibers formed from a syrup comprising an α,β unsaturated monomer having at least one pendant unit selected from a carboxylic acid group and derivatives thereof and a copolymerisable monomer. A proportion of the pendant units are preferably present in the final copolymer as the free acid and a proportion as the salt of the acid. These copolymers are capable of being cross-linked, either internally or with a variety of cross-linking agents, to form the water-absorbent composition.

Preferably before being placed in the diaper, the core may be compressed and then sprayed with a quantity of a binder, an adhesive and/or water.

Located above the water-absorbent layer is a water-management layer 4 in accordance with the first aspect of the present invention. The water-management layer 4 can be further described with reference to Figure 2. The water-management layer of Figure 2 comprises a polypropylene apertured film 41 having 130 holes per square inch. The holes are formed by the hot-needle method and therefore are of fluted cross-section. Fibers 42 are located on the surface of the apertured film. One end of each fiber is preferably bonded to the apertured film. These fibers form a high loft area.

When the water-management layer is placed on the water-absorbent layer, the base of each fluted hole preferably penetrates into the top of the water-absorbent layer. The water-management layer is preferably bonded to the water-absorbent layer.

The present invention will now be described with reference to the accompanying examples.

In the following examples, the rewet was measured by weighing 10 filter papers, placing these in contact with the cover-sheet for 15 secs under a pressure of 0.5 psi. The papers were then reweighed and the difference calculated between the weight of the papers before and after application to the coversheet. The result is a measure of the rewet.

### Example 1

A two layer core was formed from an 20 g absorbent layer comprising 5 g of superabsorbent fibers and, located above the core, a water-management layer comprising a 20 micrometer thick polypropylene sheet having 130 holes per square inch and a 50 g/m³ air through carded polyester fibrous layer. The layers were bonded together. The cover-sheet from a commercial diaper was placed onto the water-management layer. 70ml of synthetic urine were applied to the coversheet from a burette at a rate of 1 ml/s. After 20 minutes the process was repeated. After a further 20 minutes the process was again repeated. After a further 20 minutes the rewet was calculated and found to be 0.3 g.

### Example 2

A two layer core was formed from an 20 g absorbent layer comprising 5 g of superabsorbent fibers and, located above the core, a water-management layer comprising a high loft distribution layer sprayed with a silicone. The water-management layer was bonded to the absorbent core. The cover-sheet from a commercial diaper was placed onto the water-management layer. 70ml of synthetic urine were applied to the coversheet from a burette at a rate of 1 ml/s. After 20 minutes the process was repeated. After a further 20 minutes the process was again repeated. After a further 20 minutes the rewet was calculated and found to be 1 g.

### Example 3

A two layer core was formed from an 20 g absorbent layer comprising 5 g of superabsorbent fibers and, located above the core, a water-management layer comprising a hydrophobic spun bond layer of approximately 30 g/m³ having a 50 g/m³ air through carded polyester fibrous layer located above. The water-management layer was bonded to the absorbent core. The cover-sheet from a commercial diaper was placed onto the water-management layer. 70ml of synthetic urine were applied to the coversheet from a burette at a rate of 1 ml/s. After 20 minutes the process was repeated. After a further 20 minutes the process was again repeated. After a further 20 minutes the rewet was calculated and found to be 1 g.

### Comparative Example 1

An absorbent core of the type used in Examples 1 and 2 was covered with the cover-sheet from a commercial diaper. 70ml of synthetic urine were applied to the coversheet from a burette at a rate of 1 ml/s. After 20 minutes the process was repeated. After a further 20 minutes the process was again repeated. After a further 20 minutes the rewet was calculated and found to be between 10 and 20 g.

Whilst the present invention has been described with reference to sanitary napkins and pads, incontinence garments and disposable diapers it will be understood that the invention is equally applicable to other products which require high water-absorption capability such as pant liners, training pads, tampons, adult incontinence pads, bandages, patient underpads (for example pads of the type described in US 3,814,101 US 4,342,314 and EP 0 052 403), mortuary pads, casket liners, forensic examination pads, meat trays, soaker pads for food use, medical tray pads, fenestration drapes, other medical related articles, seed germination pads, capillary mats, baby bibs, desiccant strips for anti-rust use, bath mats, packaging, sorbents, clothing, breast pads, underarm pads, surgical and dental sponges, bandages, industrial wipes, domestic wipes, wipes, filters, cable wrap, food preservation articles, roofing materials, automotive trim, furniture, gasket, sealants, pond liners, bedding, clothing, cement, household pet litter, soil modifiers, wound covers and the like.

## Claims

1. A multiple layer water-absorbent core suitable for use in an absorbent article comprising a water-absorbent layer and a water-management layer, which water-management layer has an upper surface and is an apertured film or a hydrophobic non-woven material and which water-management layer is located above said waterabsorbent layer, said core **characterised in that** said water-management layer has fibers of from 15 to 50 mm in length located on its upper surface.

2. A multiple layer water-absorbent core according to claim 1 wherein the water-management layer is an apertured film formed from a hydrophobic material.

3. A multiple layer water-absorbent core according to claim 1 or 2 wherein the water-management layer is an apertured film which includes fluted holes.

4. A multiple layer water-absorbent core according to any one of the preceding claims wherein the fibers generally extend away from the water-management layer.

5. A multiple layer water-absorbent core according to any one of the preceding claims wherein voids exist between the fibers.

6. A multiple layer water-absorbent core according to any one of the preceding claims wherein the fibers are arranged such that there are grooves or channels formed between the fibers or between groups of fibers.

7. A multiple layer water-absorbent core according to any one of the preceding claims wherein the fibers are selected from rayon fibers, cellulose ester fibers, protein fibers, polyamide fibers, polyester fibers, polyvinyl fibers, polyolefin fibers, polyurethane fibers, aramid fibers, glass fibers, fibers having a hollow core, bicomponent fibers and mixtures thereof.

8. An absorbent article comprising a liquid pervious cover sheet, a liquid impervious backsheet and a multiple layer water-absorbent core according to any one of the preceding claims wherein the cover sheet is joined to the backsheet to enclose the water-absorbent core.

9. An absorbent article according to claim 8 wherein the fibers are arranged such they extended in a direction substantially parallel to the longitudinal axis of the absorbent article.

## Patentansprüche

1. Mehrschichtiger, wasserabsorbierender Kern, der für die Verwendung in einem absorbierenden Gegenstand geeignet ist, umfassend eine wasserabsorbierende Schicht und eine Wassermanagementschicht, wobei die Wassermanagementschicht eine obere Oberfläche aufweist und einen gelochten Belag oder ein hydrophobes, nichtgewebtes Material darstellt und sich die Wassermanagementschicht oberhalb der wasserabsorbierenden Schicht befindet, wobei der Kern **dadurch gekennzeichnet ist, dass** die Wassermanagementschicht Fasern mit einer Länge von 15 bis 50 mm aufweist, die sich auf deren oberer Oberfläche befinden.

2. Mehrschichtiger, wasserabsorbierender Kern nach Anspruch 1, in dem die Wassermanagementschicht ein gelochter Belag ist, der aus einem hydrophoben Material gebildet wird.

3. Mehrschichtiger, wasserabsorbierender Kern nach Anspruch 1 oder 2, in dem die Wassermanagementschicht ein gelochter Belag ist, der ausgekehlte Löcher beinhaltet.

4. Mehrschichtiger, wasserabsorbierender Kern nach einem der vorstehenden Ansprüche, in dem sich die Fasern allgemein weg von der Wassermanagementschicht erstrecken.

5. Mehrschichtiger, wasserabsorbierender Kern nach einem der vorstehenden Ansprüche, in dem Leerräume zwischen den Fasern existieren.

6. Mehrschichtiger, wasserabsorbierender Kern nach einem der vorstehenden Ansprüche, in dem die Fasern so angeordnet sind, dass es Aussparungen oder Kanäle gibt, die zwischen den Fasern oder zwischen Gruppen von Fasern gebildet werden.

7. Mehrschichtiger, wasserabsorbierender Kern nach einem der vorstehenden Ansprüche, in dem die Fasern ausgewählt werden aus Kunstseidefasern, Celluloseesterfasern, Proteinfasern, Polyamidfasern, Polyesterfasern, Polyvinylfasern, Polyolefinfasern, Polyurethanfasern, Aramidfasern, Glasfasern, Fasern mit einem Hohlkern, Zweikomponentenfasern, und Gemischen davon.

8. Absorbierender Gegenstand, umfassend eine flüssigkeitsdurchlässige Deckschicht, eine flüssigkeitsundurchlässige Rückseitenschicht und einen mehrschichtigen, wasserabsorbierenden Kern nach einem der vorstehenden Ansprüche, wobei die Deckschicht mit der Rückseitenschicht verbunden ist, um den wasserabsorbierenden Kern zu umgeben.

9. Absorbierender Gegenstand nach Anspruch 8, in dem die Fasern so angeordnet sind, dass sie sich in einer Richtung erstrecken, die im wesentlichen parallel zu der longitudinalen Achse des absorbierenden Gegenstandes ist.

## Revendications

1. Noyau absorbant l'eau à couches multiples convenant pour être utilisé dans un article absorbant comprenant une couche absorbant l'eau et une couche gérant l'eau, ladite couche gérant l'eau a une surface supérieure et est constituée d'un film perforé ou d'un matériau hydrophobe non tissé et ladite couche gérant l'eau est située au-dessus de ladite couche absorbant l'eau, ledit noyau étant **caractérisé en ce que** ladite couche gérant l'eau possède des fibres d'une longueur comprise entre 15 et 30 mm placées sur sa surface supérieure.

2. Noyau absorbant l'eau à couches multiples selon la revendication 1, dans lequel la couche gérant l'eau est un film perforé formé à partir d'un matériau hydrophobe.

3. Noyau absorbant l'eau à couches multiples selon la revendication 1 ou 2, dans lequel la couche gérant l'eau est un film perforé qui comprend des trous cannelés.

4. Noyau absorbant l'eau à couches multiples selon l'une quelconque des revendications précédentes, dans lequel les fibres s'étendent, en général, dans une direction s'éloignant de la couche gérant l'eau.

5. Noyau absorbant l'eau à couches multiples selon l'une quelconque des revendications précédentes, dans lequel il existe des interstices entre les fibres.

6. Noyau absorbant l'eau à couches multiples selon l'une quelconque des revendications précédentes, dans lequel les fibres sont disposées de telle façon que des rainures ou des canaux soient formés entre les fibres ou les groupes de fibres.

7. Noyau absorbant l'eau à couches multiples selon l'une quelconque des revendications précédentes, dans lequel les fibres sont sélectionnées parmi des fibres de rayonne, des fibres d'esters de cellulose, des fibres protéiques, des fibres de polyamide, des fibres polyester, des fibres de polyvinyle, des fibres polyoléfiniques, des fibres de polyuréthanne, des fibres aramides, des fibres de verre, des fibres ayant une âme creuse, des fibres à deux composants ou des mélanges de ces fibres.

8. Article absorbant comprenant une feuille de couverture perméable aux liquides, une feuille arrière imperméable aux liquides et un noyau absorbant l'eau à couches multiples selon l'une quelconque des revendications précédentes, dans lequel la feuille de couverture est reliée à la feuille arrière pour enceindre le noyau absorbant l'eau.

9. Article absorbant selon la revendication 8, dans lequel les fibres sont disposées de façon à s'étendre dans une direction essentiellement parallèle à l'axe longitudinal de l'article absorbant.
